(19) Europäisches Patentamt
European Patent Office
Office européen des brevets

(11) Veröffentlichungsnummer: **0 095 115**
**A1**

(12) # EUROPÄISCHE PATENTANMELDUNG

(21) Anmeldenummer: **83104785.7**

(22) Anmeldetag: **16.05.83**

(51) Int. Cl.³: **C 07 D 231/12**
C 07 D 233/60, C 07 D 317/24
C 07 D 319/06, C 07 D 249/04
C 07 D 249/08, C 07 F 7/08
C 07 F 9/09, C 07 C 69/712
C 07 F 9/165, C 07 C 69/63

(30) Priorität: **26.05.82 DE 3219821**
**31.12.82 DE 3248779**

(43) Veröffentlichungstag der Anmeldung:
**30.11.83 Patentblatt 83/48**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Förster, Heinz, Dr.**
**Am Eckbusch 47**
**D-5600 Wuppertal 1(DE)**

(72) Erfinder: **Priesnitz, Uwe, Dr.**
**Severinstrasse 58**
**D-5650 Solingen(DE)**

(72) Erfinder: **Riebel, Hans-Jochem, Dr.**
**In der Beek 92**
**D-5600 Wuppertal 1(DE)**

(72) Erfinder: **Eue, Ludwig, Dr.**
**Paul-Klee-Strasse 36**
**D-5090 Leverkusen 1(DE)**

(72) Erfinder: **Schmidt, Robert R., Dr.**
**Im Waldwinkel 110**
**D-5060 Bergisch-Gladbach 2(DE)**

(54) **Substituierte Diphenylether.**

(57) Neue substituierte Diphenylether der Formel

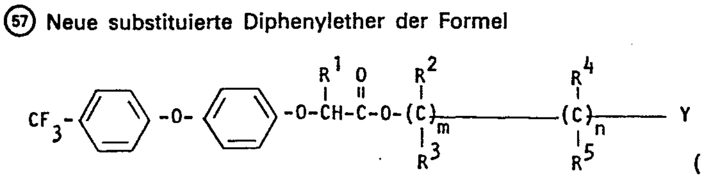

(I)

in welcher
$R^1$, $R^2$, $R^3$, $R^4$, $R^5$, Y, m und n die in der Beschreibung angegebene Bedeutung haben,
mehrere Verfahren zur Herstellung der neuen Stoffe und deren Verwendung als Herbizide.

Neue Zwischenprodukte sowie Verfahren zu deren Herstellung.

EP 0 095 115 A1

BAYER AKTIENGESELLSCHAFT 5090 Leverkusen, Bayerwerk
Zentralbereich
Patente, Marken und Lizenzen Dü/bo/c
Ib

## Substituierte Diphenylether

Die Erfindung betrifft neue substituierte Diphenylether, mehrere Verfahren zu ihrer Herstellung sowie ihre Verwendung als Herbizide.

Es ist bereits bekannt geworden, daß zahlreiche Phenoxypropionsäure-Derivate herbizide Eigenschaften besitzen (vgl. DE-OS 22 23 894 und DE-OS 27 58 002). So können zum Beispiel der 2-/4-(2,4-Dichlorphenoxy)-phenoxy/-propionsäure-methylester und das R-Enantiomere*) des 2-/4-(4-Trifluormethyl-phenoxy)-phenoxy/-propionsäure-ethylesters zur Unkrautbekämpfung eingesetzt werden. Die Wirkung dieser Stoffe ist jedoch insbesondere bei einigen Gräsern und beim Einsatz niedriger Aufwandmengen nicht immer ausreichend.

---

*) Unter R-Enantiomeren sind hier jeweils diejenigen optisch aktiven Verbindungen zu verstehen, welche am asymmetrischen Kohlenstoffatom der Propionsäure-Einheit die R-Konfiguration aufweisen.

Es wurden nun neue substituierte Diphenylether der Formel

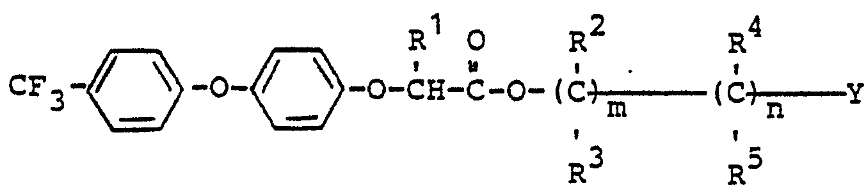 (I)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes über Stickstoff gebundenes Azolyl und die Reste der Formeln

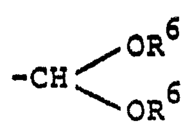 oder 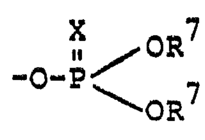 steht,

in welchen

$R^6$ für Alkyl mit 1 bis 4 Kohlenstoffatomen steht oder die beiden Reste $R^6$ gemeinsam für eine

Alkylenkette mit 2 oder 3 Kohlenstoffatomen stehen,

$R^7$ für Alkyl mit 1 bis 4 Kohlenstoffatomen steht und

X für Sauerstoff oder Schwefel steht,

gefunden.

Die substituierten Diphenylether der Formel (I), in denen $R^1$ für Methyl steht, enthalten ein asymmetrisches Kohlenstoffatom und können deshalb in zwei enantiomeren Formen vorliegen. Die Erfindung betrifft sowohl die jeweiligen Racemate als auch die R- und S-Enantiomeren.

Weiterhin wurde gefunden, daß man

a) diejenigen substituierten Diphenylether der Formel (I), in denen Y für Trimethylsilyl oder für den Rest der Formel

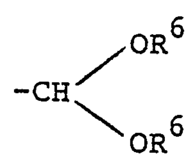

steht, erhält, wenn man Phenoxyalkancarbonsäuren der Formel

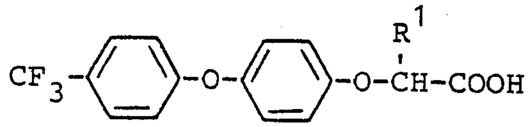

(II)

in welcher

$R^1$ die oben angegebene Bedeutung hat,

α) mit Silylchloriden der Formel

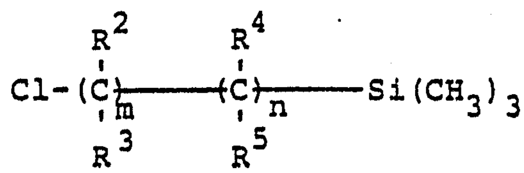

(IIIa)

in welcher

$R^2$, $R^3$, $R^4$, $R^5$, m und n die oben angegebene Bedeutung haben,

oder

β) mit Acetalen der Formel

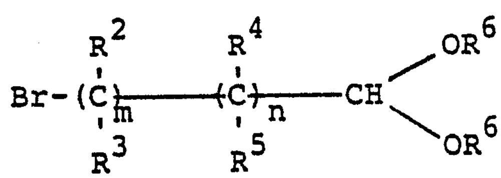

(IIIb)

in welcher

$R^2$, $R^3$, $R^4$, $R^5$, $R^6$, m und n die oben angegebene Bedeutung haben,

jeweils gegebenenfalls in Gegenwart eines Säurebindemittels sowie gegebenenfalls in Gegenwart eines Verdünnungsmittels umsetzt,

b) diejenigen substituierten Diphenylether der Formel (I),

in denen

Y für gegebenenfalls substituiertes über Stickstoff gebundenes Azolyl oder für die Reste der Formeln $-CH(OR^6)_2$ oder

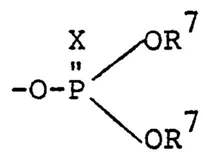

steht, erhält,

wenn man Phenoxyalkancarbonsäurechloride der Formel

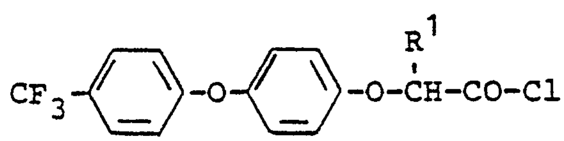

(IV)

in welcher

$R^1$ die oben angegebene Bedeutung hat,

mit Verbindungen der Formel

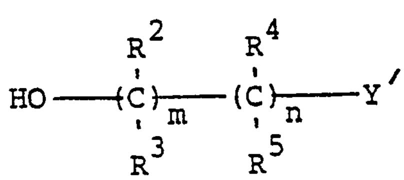

(V)

in welcher

$R^2$, $R^3$, $R^4$, $R^5$, m und n die oben angegebene Bedeutung haben und

Y' für gegebenenfalls substituiertes über Stickstoff gebundenes Azolyl, einen Rest der Formel $-CH(OR^6)_2$ oder der Formel

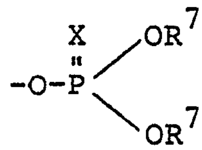

steht, wobei

$R^6$, $R^7$ und X die oben angegebene Bedeutung haben,

gegebenenfalls in Gegenwart eines Säurebindemittels sowie gegebenenfalls in Gegenwart eines Verdünnungsmittels umsetzt,

oder

c) Verbindungen der Formel (I) erhält, wenn man Benzotrifluoride der Formel

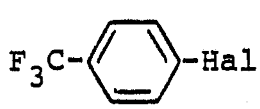

(VI)

in welcher

Hal für Chlor, Brom oder Jod steht,

mit Hydrochinon-monoethern der Formel

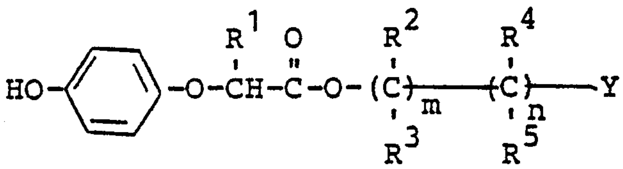

(VII)

in welcher

$R^1$, $R^2$, $R^3$, $R^4$, $R^5$, Y, m und n die oben angegebene Bedeutung haben,

gegebenenfalls in Gegenwart eines Säurebindemittels und gegebenenfalls in Gegenwart eines Verdünnungsmittels umsetzt,

oder

d) Verbindungen der Formel (I) erhält, wenn man 4-(4-Trifluormethyl-phenoxy)-phenol der Formel

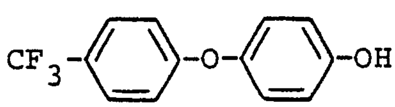

(VIII)

mit Alkancarbonsäure-Derivaten der Formel

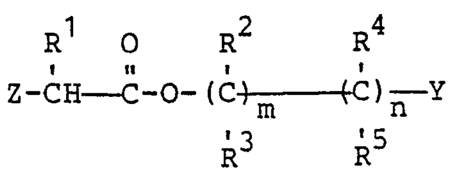

(IX)

in welcher

$R^1$, $R^2$, $R^3$, $R^4$, $R^5$, Y, m und n die oben angegebene Bedeutung haben und

Z für Chlor, Brom, Tosylat oder Mesylat steht,

gegebenenfalls in Gegenwart eines Säurebindemittels sowie gegebenenfalls in Gegenwart eines Verdünnungsmittels umsetzt.

Schließlich wurde gefunden, daß sich die neuen substituierten Diphenylether der Formel (I) durch eine hervorragende herbizide Wirksamkeit auszeichnen.

Überraschenderweise besitzen die erfindungsgemäßen substituierten Diphenylether der Formel (I) wesentlich bessere herbizide Eigenschaften als der aus dem Stand der Technik bekannte 2-[4-(2,4-Dichlorphenoxy)-phenoxy]-propionsäuremethylester, welches ein hoch wirksamer Wirkstoff gleicher Wirkungsart ist. Vor allem lassen sich mit Hilfe der erfindungsgemäßen Wirkstoffe einige Ungräser, die von dem 4-[4-(2,4-Dichlorphenoxy)-phenoxy]-propionsäure-methylester nicht voll erfaßt werden, wirksam bekämpfen.

Im übrigen übertreffen die R-Enantiomeren der erfindungsgemäßen Stoffe der Formel (I), in denen $R^1$ für Methyl steht, überraschenderweise auch das aus dem Stand der Technik bekannte, konstitutionell ähnliche

R-Enantiomere des 2-/4-(4-Trifluormethylphenoxy)-Phenoxy7-propionsäure-ethylesters bezüglich der herbiziden Eigenschaften.

Die erfindungsgemäßen substituierten Diphenylether sind durch die Formel (I) eindeutig definiert. In dieser Formel steht $R^1$ für Wasserstoff oder Methyl. $R^2$, $R^3$, $R^4$ und $R^5$ stehen unabhängig voneinander vorzugsweise für Wasserstoff oder Methyl. Der Index m steht vorzugsweise für 1 oder 2, und der Index n steht vorzugsweise für 0 oder 1. Der Substituent Y steht vorzugsweise für Trimethylsilyl oder für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest, wobei jeder dieser Azolyl-Reste ein- oder mehrfach, gleichartig oder verschieden substituiert sein kann durch Fluor, Chlor, Brom, Jod, Alkyl mit 1 bis 4 Kohlenstoffatomen, Alkoxy mit 1 bis 4 Kohlenstoffatomen und/oder Phenyl. Weiterhin steht Y vorzugsweise für die Reste der Formeln

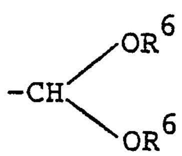 und 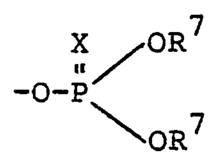 .

In diesen Resten steht $R^6$ vorzugsweise für Alkyl mit 1 oder 2 Kohlenstoffatomen, oder die beiden Substituenten $R^6$ stehen gemeinsam für eine Alkylenkette mit 2 oder 3 Kohlenstoffatomen. $R^7$ steht vorzugsweise für Alkyl mit 1 bis 3 Kohlenstoffatomen und X steht vorzugsweise für Sauerstoff oder Schwefel.

Eine besonders bevorzugte Gruppe von erfindungsgemäßen Verbindungen sind diejenigen Stoffe der Formel (I), in denen $R^1$ für Wasserstoff oder Methyl steht, $R^2$, $R^3$, $R^4$, $R^5$, m und n diejenigen Bedeutungen haben, die oben bereits vorzugsweise für diese Reste und Indices genannt wurden und Y für Trimethylsilyl steht.

Eine andere Gruppe von besonders bevorzugten erfindungsgemäßen Verbindungen sind diejenigen Stoffe der Formel (I), in denen $R^1$ für Wasserstoff oder Methyl steht, $R^2$, $R^3$, $R^4$, $R^5$, m und n diejenigen Bedeutungen haben, die oben bereits vorzugsweise für diese Reste und Indices genannt wurden, und Y für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein-, zwei- oder dreifach, gleichartig oder verschieden substituiert sein kann durch Fluor, Chlor, Brom, Alkyl mit 1 oder 2 Kohlenstoffatomen, Alkoxy mit 1 oder 2 Kohlenstoffatomen und/oder Phenyl.

Eine weitere Gruppe von besonders bevorzugten erfindungsgemäßen Verbindungen sind ferner diejenigen Stoffe der Formel (I), in denen $R^1$ für Wasserstoff oder Methyl steht, $R^2$, $R^3$, $R^4$, $R^5$, m und n diejenigen Bedeutungen haben, die oben bereits vorzugsweise für diese Reste und Indices genannt wurden, und Y für die Gruppierung der Formel

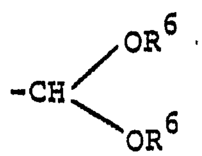

steht, in welcher die Substituenten $R^6$ diejenigen Bedeutungen haben, die oben bereits vorzugsweise für $R^6$ genannt wurden.

Eine andere Gruppe von besonders bevorzugten erfindungsgemäßen Verbindungen sind schließlich diejenigen Stoffe der Formel (I), in denen $R^1$ für Wasserstoff oder Methyl steht,

$R^2$, $R^3$, $R^4$, $R^5$, m und n diejenigen Bedeutungen haben, die oben bereits vorzugsweise für diese Reste und Indices genannt wurden, und Y für die Gruppierung der Formel

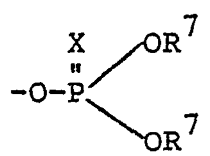

steht, in welcher die Substituenten $R^7$ und X diejenigen Bedeutungen haben, die oben bereits vorzugsweise für $R^7$ bzw. X genannt wurden.

Als Beispiele für substituierte Diphenylether der Formel (I) seien die in den folgenden Tabellen formelmäßig aufgeführten Verbindungen genannt.

Tabelle 1

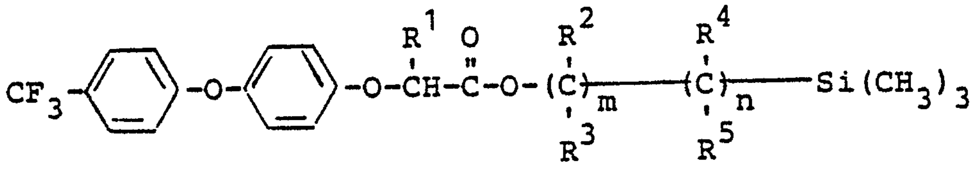 (Ia)

| $R^1$ | $R^2$ | $R^3$ | $R^4$ | $R^5$ | m | n |
|---|---|---|---|---|---|---|
| H | H | H | H | H | 1 | 1 |
| H | H | H | - | - | 1 | 0 |
| $CH_3$ | H | H | H | H | 1 | 1 |
| $CH_3$ | H | H | - | - | 1 | 0 |

Tabelle 2

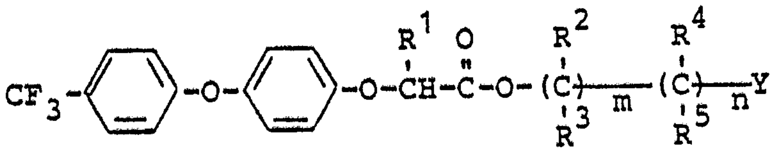 (Ib)

| $R^1$ | $R^2$ | $R^3$ | $R^4$ | $R^5$ | m | n | Y |
|---|---|---|---|---|---|---|---|
| H | H | H | - | - | 1 | 0 | 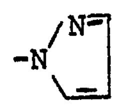 |
| $CH_3$ | H | H | - | - | 1 | 0 | 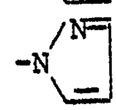 |
| H | H | H | H | H | 1 | 0 | 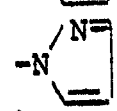 |
| $CH_3$ | H | H | H | H | 1 | 0 | 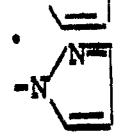 |
| H | H | H | - | - | 1 | 0 | 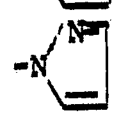 |

Tabelle 2 (Fortsetzung)

| $R^1$ | $R^2$ | $R^3$ | $R^4$ | $R^5$ | m | n | Y |
|---|---|---|---|---|---|---|---|
| $CH_3$ | H | H | H | H | 1 | 1 | 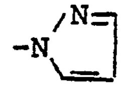 |
| $CH_3$ | H | H | - | - | 1 | 0 | 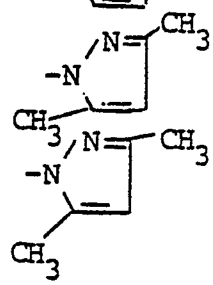 |
| $CH_3$ | H | H | H | H | 1 | 1 | |
| $CH_3$ | H | H | - | - | 1 | 0 | 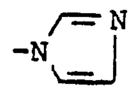 |
| $CH_3$ | H | H | H | H | 1 | 1 | 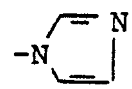 |
| $CH_3$ | H | H | - | - | 1 | 0 | 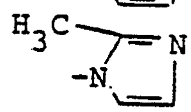 |
| $CH_3$ | H | H | H | H | 1 | 1 | 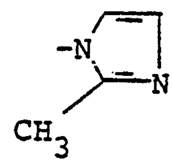 |
| $CH_3$ | H | H | - | - | 1 | 0 | 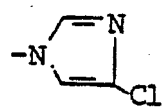 |
| $CH_3$ | H | H | - | - | 1 | 0 | 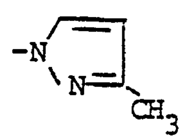 |
| $CH_3$ | H | H | - | - | 1 | 0 | 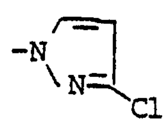 |
| $CH_3$ | H | H | - | - | 1 | 0 | 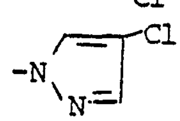 |

Tabelle 2 (Fortsetzung)

| $R^1$ | $R^2$ | $R^3$ | $R^4$ | $R^5$ | m | n | Y |
|---|---|---|---|---|---|---|---|
| $CH_3$ | H | H | - | - | 1 | 0 | 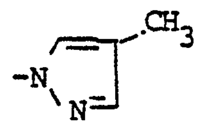 |
| $CH_3$ | H | H | - | - | 1 | 0 | 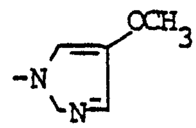 |
| $CH_3$ | H | H | - | - | 1 | 0 | 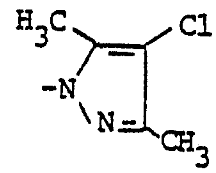 |
| $CH_3$ | H | H | $CH_3$ | $CH_3$ | 1 | 1 | 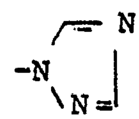 |
| $CH_3$ | H | H | $CH_3$ | $CH_3$ | 1 | 1 | 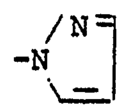 |
| H | H | H | $CH_3$ | $CH_3$ | 1 | 1 | 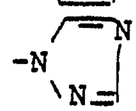 |
| H | H | H | $CH_3$ | $CH_3$ | 1 | 1 | 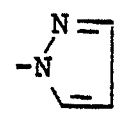 |

Tabelle 3

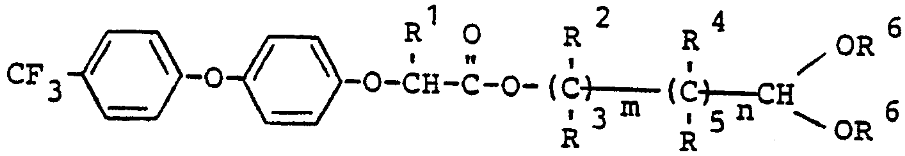

(Ic)

| $R^1$ | $R^2$ | $R^3$ | $R^4$ | $R^5$ | m | n | $R^6$ | $R^6$ |
|---|---|---|---|---|---|---|---|---|
| H | H | H | - | - | 1 | 0 | $CH_3$ | $CH_3$ |
| $CH_3$ | H | H | - | - | 1 | 0 | $CH_3$ | $CH_3$ |
| H | H | H | H | H | 1 | 1 | $CH_3$ | $CH_3$ |
| $CH_3$ | H | H | H | H | 1 | 1 | $CH_3$ | $CH_3$ |
| $CH_3$ | H | H | - | - | 1 | 0 | $C_2H_5$ | $C_2H_5$ |
| $CH_3$ | H | H | - | - | 1 | 0 | $-CH_2-CH_2-$ | |
| $CH_3$ | H | H | - | - | 1 | 0 | $-(CH_2)_3-$ | |

Tabelle 4

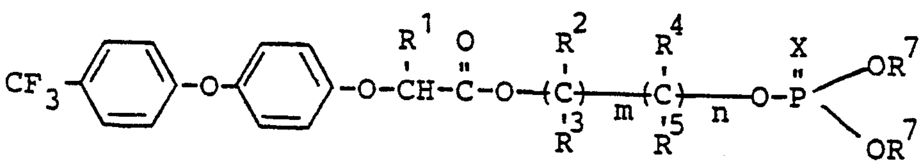

(Id)

| $R^1$ | $R^2$ | $R^3$ | $R^4$ | $R^5$ | m | n | X | $R^7$ | $R^7$ |
|---|---|---|---|---|---|---|---|---|---|
| H | H | H | H | H | 1 | 1 | O | $CH_3$ | $CH_3$ |
| $CH_3$ | H | H | H | H | 1 | 1 | O | $CH_3$ | $CH_3$ |
| H | H | H | H | H | 1 | 1 | S | $CH_3$ | $CH_3$ |
| $CH_3$ | H | H | H | H | 1 | 1 | S | $CH_3$ | $CH_3$ |
| $CH_3$ | H | H | H | H | 1 | 1 | S | $C_2H_5$ | $C_2H_5$ |
| $CH_3$ | H | H | H | H | 1 | 1 | O | $C_2H_5$ | $C_2H_5$ |
| $CH_3$ | H | H | H | H | 1 | 1 | S | $C_3H_7$-n | $C_3H_7$-n |
| $CH_3$ | H | H | H | H | 1 | 1 | O | $C_3H_7$-n | $C_3H_7$-n |

Besonders bevorzugt sind auch die R-Enantiomeren derjenigen obengenannten Stoffe der Formel (I), in denen $R^1$ für Methyl steht, also die erfindungsgemäßen Verbindungen der Formel

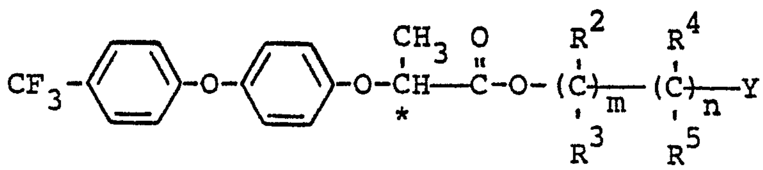
(Ie)

in welcher

$R^2$, $R^3$, $R^4$, $R^5$, Y, m und n die oben als bevorzugt genannte Bedeutung haben.

In dieser Formel ist das asymmetrische Kohlenstoffatom (Asymmetriezentrum), das die R-Konfiguration aufweist, durch ein (*) gekennzeichnet.

Verwendet man 2-/4-(4-Trifluormethyl-phenoxy)-phenoxy_7-propionsäure und Chlormethyl-trimethylsilan als Ausgangsstoffe, so kann der Verlauf des erfindungsgemäßen Verfahrens (a, Variante α) durch das folgende Formelschema wiedergegeben werden:

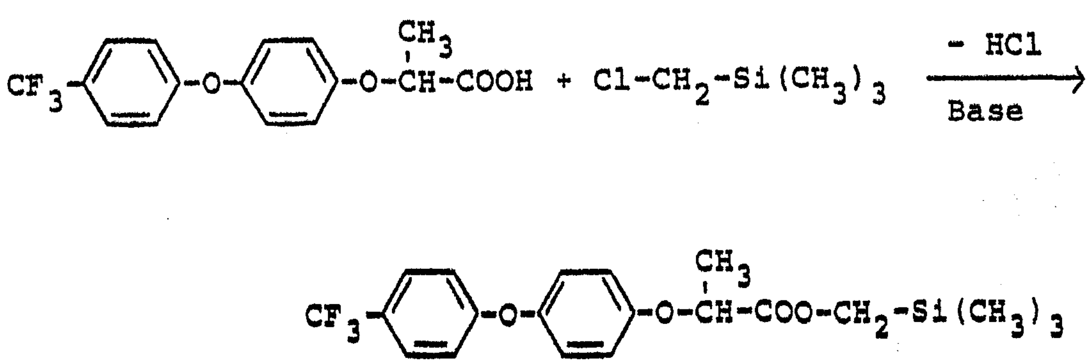

Verwendet man 2-[4-(4-Trifluormethyl-phenoxy)-phenoxy]-propionsäure und 2-Bromacetaldehyd-dimethylacetal als Ausgangsstoffe, so kann der Verlauf des erfindungsgemäßen Verfahrens (a, Variante ß) durch das folgende Formelschema wiedergegeben werden:

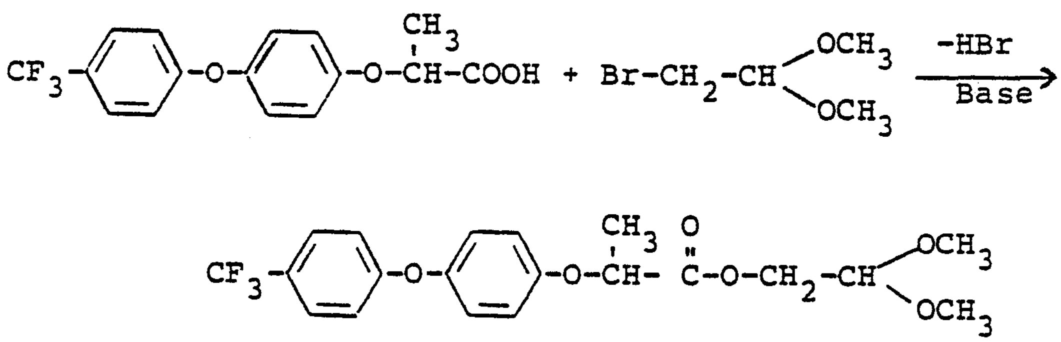


Verwendet man 2- [4-(4-Trifluormethyl-phenoxy)-phenoxy]-propionsäurechlorid und 1-Hydroxymethyl-1,2,4-triazol als Ausgangsstoffe, so kann der Verlauf des erfindungsgemäßen Verfahrens (b) durch das folgende Formelschema wiedergegeben werden:

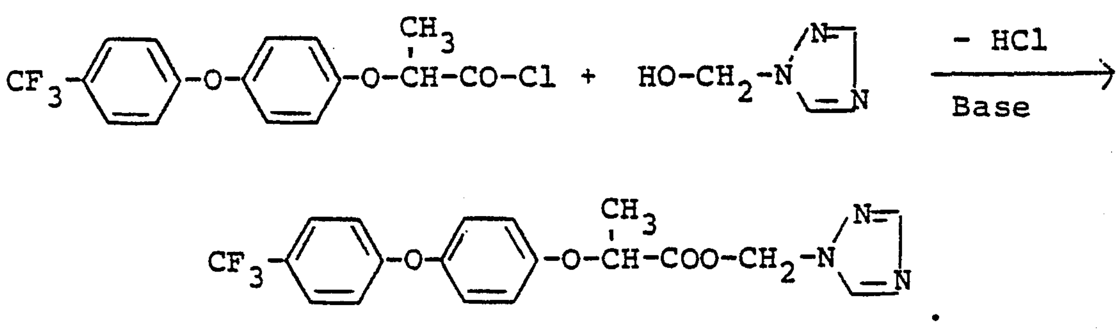

Verwendet man 4-Trifluormethyl-brombenzol und 2-(4-Hydroxy-phenoxy)-propionsäure-(pyrazol-1-yl-methyl)-ester als Ausgangsstoffe, so kann der Verlauf des erfindungsgemäßen Verfahrens (c) durch das folgende Formelschema wiedergegeben werden:

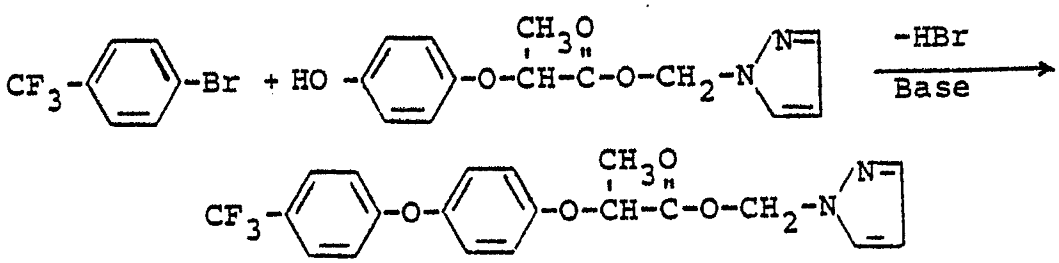


Verwendet man 4-(4-Trifluormethyl-phenoxy)-phenol und 2-Tosyloxy-propionsäure-2-(pyrazol-1-yl)-ethylester als Ausgangsstoffe, so kann der Verlauf des erfindungsgemäßen Verfahrens (d) durch das folgende Formelschema wiedergegeben werden:

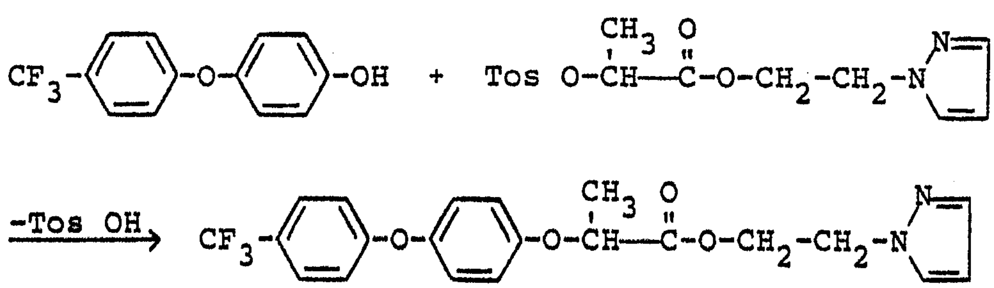


Tos = Tosyl (= 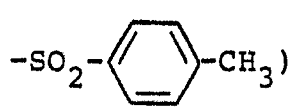

Die bei dem erfindungsgemäßen Verfahren (a) als Ausgangsstoffe benötigten Phenoxyalkancarbonsäuren sind durch die Formel (II) definiert. In dieser Formel steht $R^1$ für Wasserstoff oder Methyl.

Die Verbindungen der Formel (II) sind bekannt (vgl. DE-OS 28 02 818, DE-OS 26 46 124 und DE-OS 25 28 384).

Die bei dem erfindungsgemäßen Verfahren (a, Variante $\alpha$) weiterhin als Ausgangsstoffe benötigten Silylchloride sind durch die Formel (IIIa) definiert. In dieser Formel haben $R^2$, $R^3$, $R^4$, $R^5$, m und n vorzugsweise diejenigen Bedeutungen, die bereits im Zusammenhang mit der Beschreibung der erfindungsgemäßen Stoffe der Formel (I) vorzugsweise für diese Reste und Indices genannt wurden.

Als Beispiele für Verbindungen der Formel (IIIa) seien die in der folgenden Tabelle 5 formelmäßig aufgeführten Stoffe genannt:

Tabelle 5

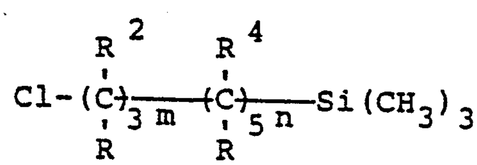

(IIIa)

| $R^2$ | $R^3$ | $R^4$ | $R^5$ | m | n |
|---|---|---|---|---|---|
| H | H | - | - | 1 | 0 |
| H | H | H | H | 1 | 1 |
| H | H | $CH_3$ | $CH_3$ | 1 | 1 |
| H | H | H | H | 2 | 1 |

Die Silylchloride der Formel (IIIa) sind bekannt oder lassen sich in einfacher Weise nach bekannten Verfahren herstellen.

Die bei dem erfindungsgemäßen Verfahren (a, Variante ß) als Ausgangsstoffe verwendbaren Acetale sind durch die Formel (IIIb) definiert. In dieser Formel haben $R^2$, $R^3$, $R^4$, $R^5$, $R^6$, m und n vorzugsweise diejenigen Bedeutungen, die bereits im Zusammenhang mit der Beschreibung der erfindungsgemäßen Stoffe der Formel (I) vorzugsweise für diese Reste und Indices genannt wurden.

**Als Beispiele für Verbindungen der Formel (IIIb) seien die in der folgenden Tabelle 6 formelmäßig aufgeführten Stoffe genannt:**

**Tabelle 6**

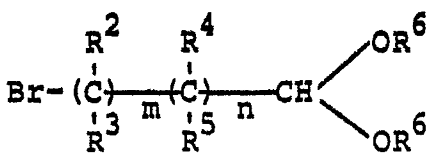

(IIIb)

| $R^2$ | $R^3$ | $R^4$ | $R^5$ | m | n | $R^6$ | $R^6$ |
|---|---|---|---|---|---|---|---|
| H | H | - | - | 1 | 0 | $CH_3$ | $CH_3$ |
| H | H | H | H | 1 | 1 | $CH_3$ | $CH_3$ |
| H | H | - | - | 1 | 0 | $C_2H_5$ | $C_2H_5$ |
| H | H | H | H | 1 | 1 | $C_2H_5$ | $C_2H_5$ |
| H | H | - | - | 1 | 0 | $-CH_2-CH_2-$ | |
| H | H | H | H | 1 | 1 | $-CH_2-CH_2-$ | |
| H | H | - | - | 1 | 0 | $-(CH_2)_3-$ | |
| H | H | H | H | 1 | 1 | $-(CH_2)_3-$ | |

Die Verbindungen der Formel (IIIb) sind bekannt oder lassen sich in einfacher Weise nach bekannten Methoden herstellen.

Zur Herstellung von R- und S-Enantiomeren der substituierten Diphenylether der Formel (I), in denen $R^1$ für Methyl steht, werden bei der Durchführung des erfindungsgemäßen Verfahrens (a) optisch aktive R- oder S-Phenoxy-propionsäure der Formel (II) als Ausgangsstoffe benötigt. Auch diese optisch aktiven Phenoxy-propionsäuren sind bekannt (vgl. DE-OS 27 58 002).

Man erhält das R- bzw. S-Enantiomere der Phenoxypropionsäure der Formel

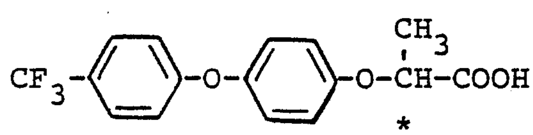

(IIa)

indem man 4-(4-Trifluormethyl-phenoxy)-phenol der Formel

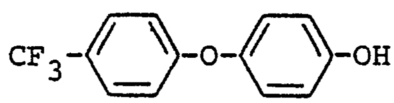

(VIII)

mit den S-Enantiomeren bzw. R-Enantiomeren von Propionsäure-Derivaten der Formel

$$\underset{*}{Z-\overset{CH_3}{\overset{|}{C}}H-COOR} \qquad (X)$$

in welcher

R für Methyl oder Ethyl steht und

Z die oben angegebene Bedeutung hat,

gegebenenfalls in Gegenwart eines Säureakzeptors, wie z.B. Kaliumcarbonat, und gegebenenfalls in Gegenwart eines Verdünnungsmittels, wie zum Beispiel Acetonitril, bei Temperaturen zwischen 0°C und 120°C umsetzt und die dabei entstehenden Ester der Formel

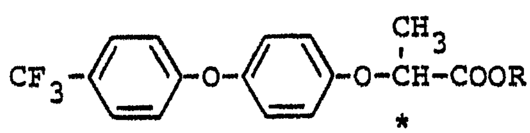

(XI)

in welcher

R die oben angegebene Bedeutung hat, mit starken Basen wie zum Beispiel Natriumhydroxid, in Gegenwart eines Verdünnungsmittels, wie zum Beispiel Methanol, Ethanol, Benzol, Toluol, Xylol, gegebenenfalls im Gemisch mit Wasser bei Temperaturen zwischen 20°C und 140°C verseift und anschließend mit einer Säure, wie zum Beispiel Salzsäure, ansäuert.

In der ersten Stufe dieser Umsetzung findet am asymmetrischen Kohlenstoffatom der Propionsäure-Einheit eine Walden'sche Umkehr statt.

Dieses hat zur Folge, daß durch Umsetzung von 4-(4-Trifluormethyl-phenoxy)-phenol der Formel (VIII) mit den S-Enantiomeren der Propionsäure-Derivate der Formel (X) das R-Enantiomere der Phenoxypropionsäure der Formel (IIa) entsteht. Andererseits bildet sich durch Umsetzung von Phenol der Formel (VIII) mit den R-Enantiomeren der Propionsäure-Derivate der Formel (X) das S-Enantiomere der Phenoxypropionsäure der Formel (IIa).

Die bei dem erfindungsgemäßen Verfahren (b) als Ausgangsstoffe benötigten Phenoxyalkancarbonsäurechloride sind durch die Formel (IV) definiert. In dieser Formel steht $R^1$ für Wasserstoff oder Methyl.

Die Phenoxyalkancarbonsäurechloride der Formel (IV) sind bekannt (vgl. DE-OS 26 28 384).

Die bei dem erfindungsgemäßen Verfahren (b) weiterhin als Ausgangsstoffe benötigten Verbindungen sind durch die Formel (V) definiert. In dieser Formel haben $R^2$, $R^3$, $R^4$, $R^5$, m und n vorzugsweise diejenigen Bedeutungen, die bereits im Zusammenhang mit der Beschreibung der erfindungsgemäßen Stoffe der Formel (I) vorzugsweise für diese Reste und Indices genannt wurden. Y' steht für einen gegebenenfalls substituierten über ein Ring-Stickstoffatom gebundenen Azolylrest, oder für einen Rest der Formel $-CH(OR^6)_2$ oder

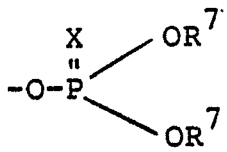

Hierbei sind unter Azolyl-Resten vorzugsweise diejenigen Azolyl-Reste zu verstehen, die bereits im Zusammenhang mit der Beschreibung der erfindungsgemäßen Stoffe der Formel (I) vorzugsweise genannt wurden. Die Substituenten $R^6$, $R^7$ und X haben vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Azolyl-Verbindungen der Formel (V) seien im einzelnen genannt:

1-Hydroxymethyl-pyrazol, 1-(1-Hydroxyethyl)-pyrazol, 1-Hydroxymethylimidazol, 1-(1-Hydroxyethyl)-imidazol, 1-Hydroxymethyl-1,2,4-triazol, 1-(1-Hydroxyethyl)-1,2,4-triazol, 1-Hydroxymethyl-1,3,4-triazol, 1-(1-Hydroxyethyl)-1,3,4-triazol, 1-Hydroxymethyl-3,5-dimethyl-pyrazol, 1-Hydroxymethyl-2-methyl-imidazol, 1-Hydroxymethyl-3-methyl-pyrazol, 3-Chlor-1-hydroxymethyl-1,2,4-triazol, 4-Chlor-1-hydroxymethyl-pyrazol, 1-Hydroxymethyl-4-methyl-pyrazol, 1-Hydroxymethyl-4-methoxy-pyrazol, 4-Chlor-1-hydroxymethyl-3,5-dimethyl-pyrazol, 1-(2-Hydroxyethyl)-pyrazol, 1-(2-Hydroxyethyl)-imidazol und 1-(1-Hydroxy-1,1-dimethyl-ethyl)-1,2,4-triazol.

Die Azolyl-Verbindungen der Formel (V) sind bereits bekannt (vgl. DE-OS 2 835 157 und DE-OS 28 35 158).

Als Beispiele für diejenigen Verbindungen der Formel (V) in denen Y' für die Reste der Formeln $-CH(OR^6)_2$ oder

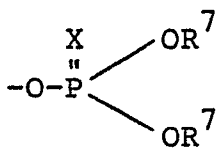

steht, seien die in den folgenden Tabellen 7 und 8 formelmäßig aufgeführten Stoffe genannt:

Tabelle 7

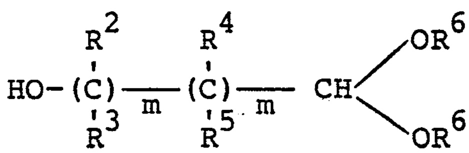

(Va)

| $R^2$ | $R^3$ | $R^4$ | $R^5$ | m | n | $R^6$ | $R^6$ |
|---|---|---|---|---|---|---|---|
| H | H | - | - | 1 | 0 | $CH_3$ | $CH_3$ |
| H | H | H | H | 1 | 1 | $CH_3$ | $CH_3$ |
| H | H | - | - | 1 | 0 | $C_2H_5$ | $C_2H_5$ |
| H | H | H | H | 1 | 1 | $C_2H_5$ | $C_2H_5$ |
| H | H | H | H | 1 | 1 | $C_2H_5$ | $C_2H_5$ |
| H | H | - | - | 1 | 0 | $-CH_2-CH_2-$ | |
| H | H | H | H | 1 | 1 | $-CH_2-CH_2-$ | |
| H | H | - | - | 1 | 0 | $-(CH_2)_3-$ | |
| H | H | H | H | 1 | 1 | $-(CH_2)_3-$ | |

Die Verbindungen der Formel (Va) sind bekannt oder lassen sich in einfacher Weise nach bekannten Methoden herstellen.

Tabelle 8

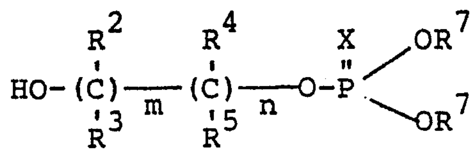

(Vb)

| $R^2$ | $R^3$ | $R^4$ | $R^5$ | m | n | X | $R^7$ | $R^7$ |
|---|---|---|---|---|---|---|---|---|
| H | H | H | H | 1 | 1 | O | $CH_3$ | $CH_3$ |
| H | H | H | H | 1 | 1 | S | $CH_3$ | $CH_3$ |
| H | H | H | H | 1 | 1 | O | $C_2H_5$ | $C_2H_5$ |
| H | H | H | H | 1 | 1 | S | $C_2H_5$ | $C_2H_5$ |
| H | H | H | H | 1 | 1 | O | $C_3H_7$-n | $C_3H_7$-n |
| H | H | H | H | 1 | 1 | S | $C_3H_7$-n | $C_3H_7$-n |

Auch die Verbindungen der Formel (Vb) sind bekannt oder lassen sich in einfacher Weise nach bekannten Methoden herstellen.

Zur Herstellung der R- und S-Enantiomeren der substituierten Diphenylether der Formel (I), in denen $R^1$ für Methyl steht, werden bei der Durchführung des erfindungsgemäßen Verfahrens (b) optisch aktives R- oder S-Phenoxypropionsäurechlorid der Formel

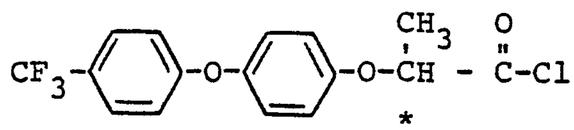

(IVa)

als Ausgangsstoffe benötigt. Letztere lassen sich aus der jeweils zugrunde liegenden Säure nach üblichen Methoden, zum Beispiel durch Chlorierung mit Thionylchlorid, herstellen (vgl. DE-OS 27 58 002).

Die bei dem erfindungsgemäßen Verfahren (c) als Ausgangsstoffe benötigten Benzotrifluoride sind durch die Formel (VI) definiert. In dieser Formel steht Hal für Chlor, Brom oder Jod. Die Benzotrifluoride der Formel (VI) sind bekannt.

Die bei dem erfindungsgemäßen Verfahren (c) weiterhin als Ausgangsstoffe benötigten Hydrochinon-monoether sind durch die Formel (VII) definiert. In dieser Formel haben $R^1$, $R^2$, $R^3$, $R^4$, $R^5$, Y, m und n vorzugsweise diejenigen Bedeutungen, die bereits im Zusammenhang mit der Beschreibung der erfindungsgemäßen Stoffe der Formel (I) vorzugsweise für diese Reste und Indices genannt wurden.

Die Hydrochinon-monoether der Formel (VII) sind bisher noch nicht bekannt. Sie lassen sich jedoch in einfacher Weise herstellen, indem man Hydrochinone der Formel

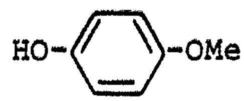

(XII)

in welcher

Me für Wasserstoff, Natrium, Kalium oder ein Äquivalent eines Calciummetalles steht,

mit Alkancarbonsäure-Derivaten der Formel

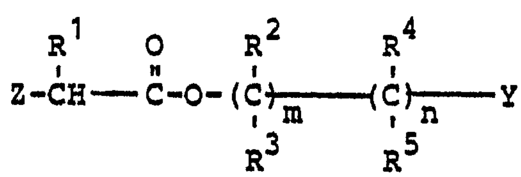

(IX)

in welcher

$R^1$, $R^2$, $R^3$, $R^4$, $R^5$, Y, Z, m und n die oben angegebene Bedeutung haben,

gegebenenfalls in Gegenwart eines Säurebindemittels sowie gegebenenfalls in Gegenwart eines polaren Verdünnungsmittels, wie zum Beispiel Dimethylformamid, Dimethylsulfoxid oder Acetonitril, bei Temperaturen zwischen 40 und 120°C, vorzugsweise zwischen 60 und 100°C, umsetzt.

Als Säureakzeptoren können bei dieser Umsetzung vorzugsweise alle diejenigen Säurebindemittel eingesetzt werden, die im Zusammenhang mit der Beschreibung der

erfindungsgemäßen Verfahren (a) bis (d) vorzugsweise als Säureakzeptoren genannt werden (vgl. unten).

Die bei dem obigen Verfahren zur Herstellung von Hydrochinon-monoethern der Formel (VII) als Ausgangsstoffe benötigten Alkancarbonsäure-Derivate der Formel (IX) lassen sich herstellen, indem man Verbindungen der Formel

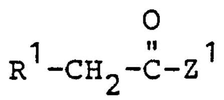

(XIII)

in welcher

$R^1$ die oben angegebene Bedeutung hat und

$Z^1$ für Hydroxy oder Chlor steht,

mit Verbindungen der Formeln

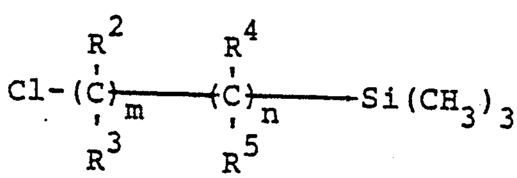

(IIIa)

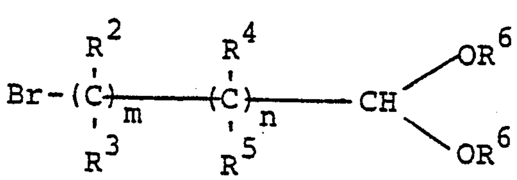

(IIIb)

oder

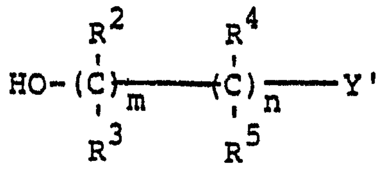 (V)

in welchen

$R^2$, $R^3$, $R^4$, $R^5$, $R^6$, Y', m und n die oben angegebenen Bedeutungen haben,

gegebenenfalls in Gegenwart eines Säurebindemittels sowie gegebenenfalls in Gegenwart eines Verdünnungsmittels umsetzt und die dabei entstehenden Stoffe der Formel

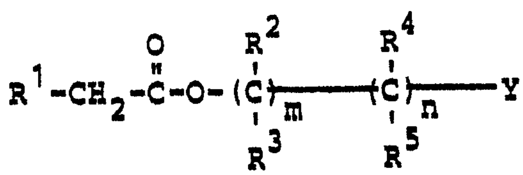 (XIV)

in welcher

$R^1$, $R^2$, $R^3$, $R^4$, $R^5$, Y, m und n die oben angegebenen Bedeutungen haben,

nach üblichen Methoden in die Alkancarbonsäure-Derivate der Formel (IX) überführt. Die Umsetzungsbedingungen bei dem obigen Verfahren zur Herstellung der Stoffe der Formel (XIV) entsprechen denjenigen des Verfahrens (a) (vgl. unten).

Es ist jedoch auch möglich, Alkancarbonsäure-Derivate der Formel (IX) herzustellen, indem man Verbindungen der Formel

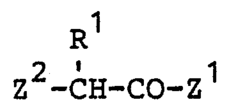

(XV)

in welcher

$R^1$ und $Z^1$ die oben angegebene Bedeutung haben und

$Z^2$ für Mesylat oder Tosylat steht,

mit Verbindungen der Formeln

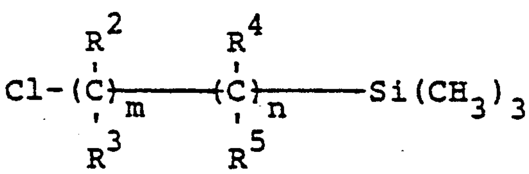

(IIIa)

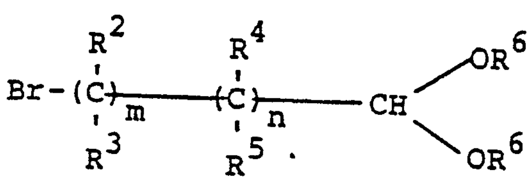

(IIIb)

oder

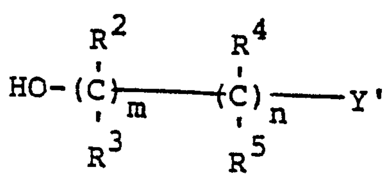

(V)

in welchen

$R^2$, $R^3$, $R^4$, $R^5$, $R^6$, Y', m und n die oben angegebenen Bedeutungen haben,

gegebenenfalls in Gegenwart eines Säurebindemittels sowie gegebenenfalls in Gegenwart eines Verdünnungsmittels umsetzt. Die Reaktionsbedingungen bei dem obigen Verfahren zur Herstellung der Stoffe der Formel (IX) entsprechen denjenigen des erfindungsgemäßen Verfahrens (a) (vgl. unten).

Die obige Methode eignet sich insbesondere zur Herstellung optisch aktiver Verbindungen der Formel (IX), also derjenigen Stoffe der Formel (IX), in denen $R^1$ für Methyl steht. Dabei entstehen aus den S-Enantiomeren der Verbindungen der Formel (XV) die S-Enantiomeren der Alkancarbonsäure-Derivate der Formel (IX). Entsprechend entstehen aus den R-Enantiomeren der Verbindungen der Formel (XV) die R-Enantiomeren der Alkancarbonsäure-Derivate der Formel (IX). Eine Walden'sche Umkehr findet im Verlauf dieser Umsetzung also nicht statt.

Zur Herstellung der R- und S-Enantiomeren der substituierten Diphenylether der Formel (I), in denen $R^1$ für Methyl steht, werden bei der Durchführung des erfindungsgemäßen Verfahrens (c) jeweils diejenigen optisch aktiven Verbindungen der Formel (VII) als Ausgangsstoffe eingesetzt, die am asymmetrischen Kohlenstoffatom bereits die gewünschte Konfiguration besitzen.

Das bei dem erfindungsgemäßen Verfahren (d) als Ausgangsmaterial benötigte 4-(4-Trifluormethyl-phenoxy)-phenol der Formel (VIII) ist bereits bekannt (vgl. DE-OS 27 58 002).

Die bei dem erfindungsgemäßen Verfahren (d) weiterhin als Ausgangsstoffe benötigten Alkancarbonsäure-Derivate sind durch die Formel (IX) definiert. In dieser Formel haben $R^1$, $R^2$, $R^3$, $R^4$, Y, m und n vorzugsweise diejenigen Bedeutungen, die bereits im Zusammenhang mit der Beschreibung der erfindungsgemäßen Stoffe der Formel (I) vorzugsweise genannt wurden. Z steht für Chlor, Brom, Tosylat oder Mesylat.

Die Herstellung der Alkancarbonsäure-Derivate der Formel (IX), die bisher noch nicht bekannt sind, wurde oben bereits beschrieben.

Wenn nach dem erfindungsgemäßen Verfahren (d) die Synthese optisch aktiver Verbindungen der Formel (I) vorgenommen werden soll, ist der Einsatz optisch aktiver Alkancarbonsäure-Derivate der Formel (IX) erforderlich. Dabei werden jeweils diejenigen Enantiomeren der Alkancarbonsäure-Derivate der Formel (IX) eingesetzt, die am asymmetrischen Kohlenstoffatom eine Konfiguration besitzen, die der im Endprodukt gewünschten Konfiguration entgegengesetzt ist. Im Verlauf der Umsetzung findet nämlich eine Walden'sche Umkehr statt.

Die erfindungsgemäßen Verfahren (a), (b), (c) und (d) zur Herstellung der neuen substituierten Diphenylether der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon sowie Dimethylsulfoxid, Tetramethylensulfon und Hexamethylphosphorsäuretriamid.

Als Säurebindemittel können sowohl bei den erfindungsgemäßen Verfahren (a), (b) und (c) als auch bei dem Verfahren (d) alle üblicherweise für derartige Umsetzungen verwendbaren Säurebindemittel eingesetzt werden. Vorzugsweise in Frage kommen Alkalihydroxide, wie z.B. Natrium- und Kaliumhydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diaza-bicyclo-[4.3.0]-nonen-5(DBN) und 1,8-Diazabicyclo-[5.4.0]-undec-7-en (DBU).

Die Reaktionstemperaturen können sowohl bei dem erfindungsgemäßen Verfahren (a) als auch bei den Verfahren (b), (c) und (d) innerhalb eines größeren Bereiches variiert werden. Im allgemeinen arbeitet man jeweils bei Temperaturen zwischen -20°C und +160°C, vorzugsweise zwischen 0°C und 140°C.

Die erfindungsgemäßen Verfahren (a), (b), (c) und (d) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c) und (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c) und (d) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive

Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können in dikotylen Kulturen sowie in Getreide und Reis zur selektiven Unkrautbekämpfung eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z.B. Erdölfraktionen, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser und mineralische oder pflanzliche Öle.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate sowie Ammoniumsalze; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azofarbstoffe, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Für die Mischungen kommen bekannte Herbizide wie zum Beispiel 1-Amino-6-ethylthio-3-(2,2-dimethylpropyl)-1,3,5-triazin-2,4-(1H, 3H)-dion oder N-(2-Benzthiazolyl)-N,N'-dimethyl-harnstoff zur Unkrautbekämpfung in Getreide, 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)on zur Unkrautbekämpfung in Sojabohnen in Frage. Einige dieser Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

## Herstellungsbeispiele

### Beispiel 1

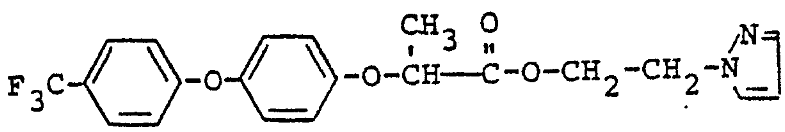

Eine Lösung von 34,5 g (0,1 Mol) 2-[4-(4-Trifluormethyl-phenoxy)-phenoxy]-propionsäurechlorid in 50 ml Toluol wurde bei 0 bis 5°C unter Rühren in eine Lösung von 11,2 g (0,1 Mol) 1-(2-Hydroxyethyl)-pyrazol und 10,6 g (0,105 Mol) Triethylamin in 100 ml Toluol gegeben. Man rührte weitere 16 Stunden bei Raumtemperatur und arbeitete dann auf, indem man das Reaktionsgemisch zunächst mit Toluol versetzte, dann mit Wasser wusch, trocknete und unter vermindertem Druck einengte. Der verbleibende Rückstand wurde durch leichtes Erwärmen im Hochvakuum von Resten an flüchtigen Anteilen befreit. Man erhielt auf diese Weise 34,9 g (83,1 % der Theorie) an 2-[4-(4-Trifluormethyl-phenoxy)-phenoxy]-propionsäure-2-(pyrazol-1-yl)-ethylester in Form eines farblosen Feststoffes vom Schmelzpunkt 65°C.

### Beispiel 2

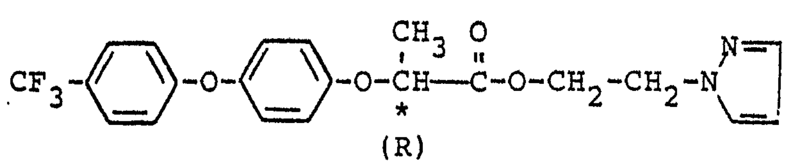

Eine Lösung von 8,6 g (0,025 Mol) des R-Enantiomeren des 2-[4-(4-Trifluormethyl-phenoxy)-phenoxy]-propion-

säurechlorids in 50 ml Toluol wurde bei 0 - 5°C unter Rühren in eine Lösung von 2,8 g (0,025 Mol) 1-(2-Hydroxyethyl)-pyrazol und 2,8 g (0,0275 Mol) Triethylamin in 100 ml Toluol gegeben. Man rührte weitere 16 Stunden bei Raumtemperatur und arbeitete dann auf, indem man das Reaktionsgemisch mit Wasser versetzte, die organische Phase nacheinander mit verdünnter wäßriger Salzsäure und Wasser wusch, dann trocknete und unter vermindertem Druck einengte. Der verbleibende Rückstand wurde durch leichtes Erwärmen im Hochvakuum von Resten an flüchtigen Anteilen befreit. Man erhielt auf diese Weise 8,2 g (78,1 % der Theorie) an dem R-Enantiomeren des 2-/4-(4-Trifluormethyl-phenoxy)-phenoxy7-propionsäure-2-(pyrazol-1-yl)-ethylesters in Form eines Feststoffes vom Schmelzpunkt 43°C.

Drehwert: $[\alpha]_D^{24}$ = + 8,1°
(1-molare Lösung in Chloroform; Küvettenlänge 10 cm).

In analoger Weise erhielt man die in den Beispielen 3 bis 6 formelmäßig aufgeführten Verbindungen der Formel (I).

Beispiel 3

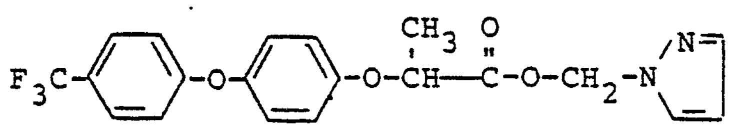

Ausbeute: 81 % der Theorie
$n_D^{21,5}$ = 1,5280

Beispiel 4

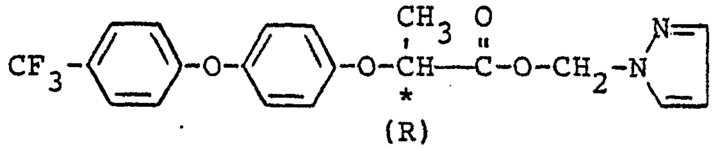

Ausbeute: 74 % der Theorie
$n_D^{24} = 1{,}5266$

Drehwert: $[\alpha]_D^{24} = + 5{,}5°$
(1-molare Lösung in Chloroform;
Küvettenlänge 10 cm).

Beispiel 5

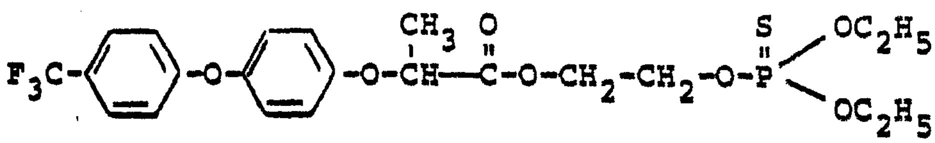

Ausbeute: 81,6 % der Theorie
$n_D^{21,5} = 1{,}5056$

Beispiel 6

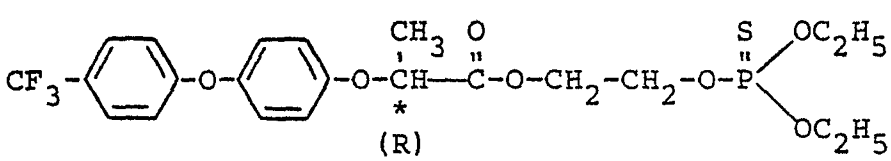

Ausbeute: 80,5 % der Theorie
Brechungsindex: $n_D^{24} = 1{,}5073$
Drehwert: $[\alpha]_D^{24} = + 6{,}5°$
(1-molare Lösung in Chloroform;
Küvettenlänge 10 cm)

Beispiel 7

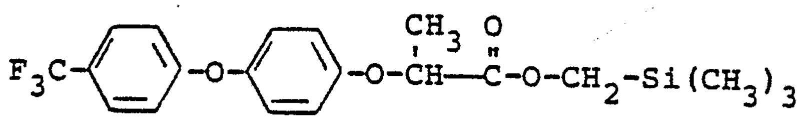

Eine Lösung von 16,3 g (0,05 Mol) 2-/4-(4-Trifluormethyl-phenoxy)-phenoxy/-propionsäure in 150 ml Aceton wurde bei 20°C unter Rühren mit 10,7 g (0,071 Mol) an 1,8-Diaza-bicyclo/5,4,0/undec-7-en ("DBU") versetzt. Das Reaktionsgemisch, in dem sich ein Niederschlag bildete, wurde weitere 30 Minuten bei Raumtemperatur gerührt und dann bei 20°C mit 8,7 g (0,071 Mol) Chlormethyl-trimethyl-silan versetzt. Das Reaktionsgemisch wurde 16 Stunden unter Rückfluß gekocht, dann in Wasser gegossen und mit konzentrierter, wäßriger Natronlauge auf pH 9-10 eingestellt. Man extrahierte mehrfach mit Toluol, wusch die organische Phase mit Wasser, trocknete und zog das Lösungsmittel unter vermindertem Druck ab. Auf diese Weise erhielt man 15 g (73 % der Theorie) an 2-/4-(4-Trifluormethyl-phenoxy)-phenoxy/-propionsäure-(trimethyl-silyl)-methylester in Form eines gelblichen Öles, das beim Animpfen durchkristallisierte.
Schmelzpunkt: 52°C.

Beispiel 8

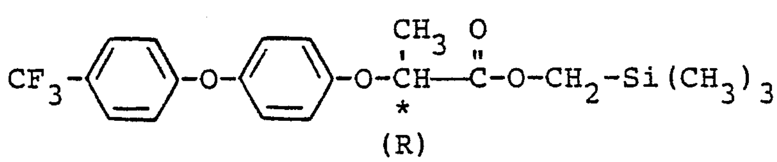

Eine Lösung von 8,2 g (0,025 Mol) des R-Enantiomeren der 2-/4-(Trifluormethyl-phenoxy)-phenoxy/-propionsäure in

50 ml Aceton wurde bei 20°C unter Rühren mit 4,9 g (0,03 Mol) 1,8-Diaza-bicyclo/5.4.0/undec-7-en ("DBU") versetzt. Das Reaktionsgemisch, in dem sich ein Niederschlag bildete, wurde 30 Minuten bei Raumtemperatur gerührt und dann bei 20°C mit 3,7 g (0,03 Mol) Chlormethyl-trimethylsilan versetzt. Man erhitzte 23 Stunden unter Rückfluß, fügte dann weitere 0,6 g Chlormethyl-trimethyl-silan und 0,7 g 1,8-Diaza-bicyclo/5.4.0/undec-7-en hinzu und erhitzte weitere 23 Stunden unter Rückfluß. Anschließend wurde aufgearbeitet, indem man das Reaktionsgemisch durch Abziehen des Lösungsmittels einengte, den verbleibenden Rückstand in Methylenchlorid aufnahm, die organische Phase nacheinander mit verdünnter wäßriger Natronlauge, verdünnter wäßriger Salzsäure und Wasser wusch, dann trocknete und das Lösungsmittel unter vermindertem Druck abzog. Der verbleibende Rückstand wurde durch leichtes Erwärmen im Hochvakuum von Resten an flüchtigen Anteilen befreit. Man erhielt auf diese Weise 7,2 g (69,9 % der Theorie) des R-Enantiomeren des 2-/4-(4-Trifluormethyl-phenoxy)-phenoxy/-propionsäure-(trimethylsilyl)-methylesters in Form eines gelblichen Öles.

Brechungsindex: $n_D^{23,5} = 1,5000$
Drehwert: $[\alpha]_D^{24} = + 7,8°$
(1-molare Lösung in Chloroform; Küvettenlänge 10 cm).

In analoger Weise erhielt man die im Beispiel 9 formelmäßig aufgeführte Verbindung.

Beispiel 9

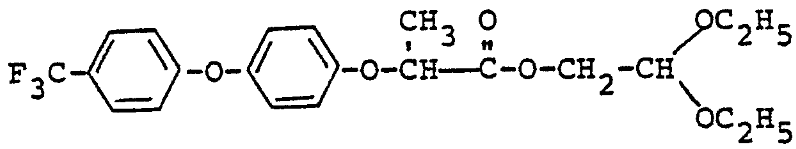

Ausbeute: 64,7 % der Theorie

$n_D^{21,5} = 1,4917$

Herstellung von Ausgangsprodukten

Beispiel 10

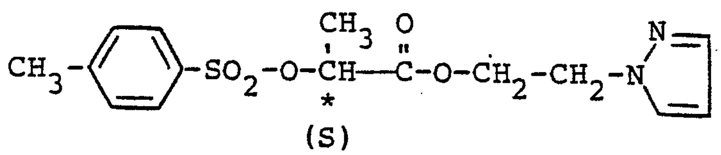

(IX-1)

5,25 g (0,02 Mol) des S-Enantiomeren des Milchsäurechlorid-tosylats wurden bei 20°C unter Rühren in ein Gemisch aus 2,24 g (0,02 Mol) 1-(2-Hydroxyethyl)-pyrazol; 2 g (0,02 Mol) Triethylamin und 50 ml Toluol gegeben. Man rührte weitere 14 Stunden bei 20°C und arbeitete dann auf, indem man das Reaktionsgemisch mit Wasser versetzte, dann mehrfach mit Toluol extrahierte, die vereinigten organischen Phasen trocknete und durch Abziehen des Lösungsmittels unter vermindertem Druck einengte. Man erhielt auf diese Weise 4,7 g (69,5 % der Theorie an dem S-Enantiomeren des 2-Tosyloxy-propionsäure-2-(pyrazol-1-yl)-ethylesters.

Drehwert: $[\alpha]_D^{24} = -11,4°$
(1-molare Lösung in Chloroform;
Küvettenlänge 10 cm).

Beispiel 11

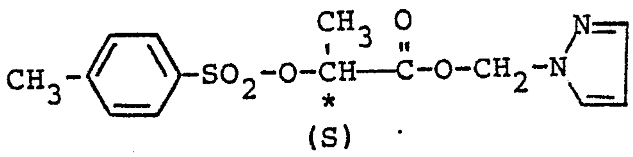

(IX-2)

Nach der im Beispiel 10 angegebenen Methode wurde auch die Verbindung der oben aufgeführten Formel hergestellt.

Ausbeute: 60 % der Theorie
Drehwert: $[\alpha]_D^{24}$ = -13,7°
(1-molare Lösung in Chloroform; Küvettenlänge 10 cm).

Beispiel 12

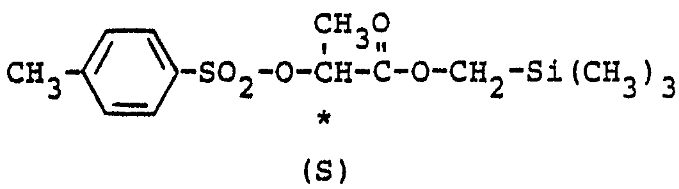

(IX-3)

Nach der im Beispiel 10 angegebenen Methode wurde auch die Verbindung der oben aufgeführten Formel hergestellt.

Ausbeute: 76 % der Theorie
Drehwert: $[\alpha]_D^{24}$ = -11,4°
(1-molare Lösung in Chloroform; Küvettenlänge 10 cm).

In den nachstehend beschriebenen biologischen Tests wurde die folgende Verbindung als Vergleichssubstanz eingesetzt:

(A) = 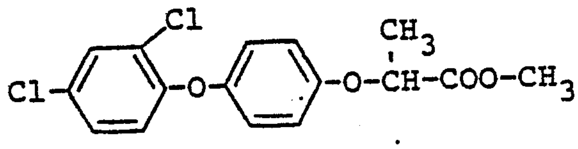

2-/4-(2,4-Dichlorphenoxy)-phenoxy_7-propionsäure-methylester
(bekannt aus DE-OS 22 23 894).

Beispiel A

Post-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (5) und (7) eine bessere selektive herbizide Wirksamkeit als die Vergleichssubstanz (A).

Patentansprüche

1. Substituierte Diphenylether der Formel

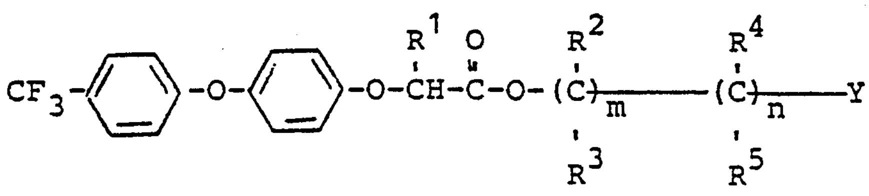 (I)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes, über Stickstoff gebundenes Azolyl und die Reste der Formeln

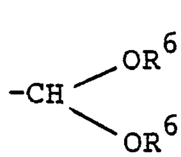 oder 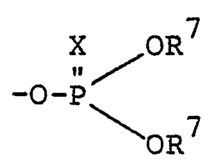 steht,

in welchen

$R^6$ für Alkyl mit 1 bis 4 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 4 Kohlenstoffatomen steht und

X für Sauerstoff oder Schwefel steht.

2. Substituierte Diphenylether der Formel (I), in denen

$R^1$ für Wasserstoff oder Methyl steht,

$R^2$, $R^3$, $R^4$ und $R^5$ unabhängig voneinander für Wasserstoff oder Methyl stehen,

m für 1 oder 2 steht,

n für 0 oder 1 steht und

Y für Trimethylsilyl oder für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl Reste ein- oder mehrfach, gleichartig oder verschieden substituiert sein kann durch Fluor, Chlor, Brom, Jod, Alkyl mit 1 bis 4 Kohlenstoffatomen, Alkoxy mit 1 bis 4 Kohlenstoffatomen und/oder Phenyl, oder

Y für einen Rest der Formel 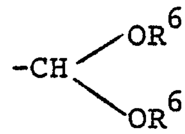 oder

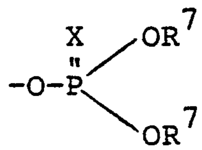.

steht, worin

$R^6$ für Alkyl mit 1 bis 2 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 3 Kohlenstoffatomen steht und

X für Sauerstoff oder Schwefel steht.

3. R-Enantiomere von substituierten Diphenylethern der Formel (I), in denen $R^1$ für Methyl steht, $R^2$, $R^3$, $R^4$ und $R^5$ unabhängig voneinander für Wasserstoff oder Methyl stehen,

m für 1 oder 2 steht,

n für 0 oder 1 steht und

Y für Trimethylsilyl oder für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden substituiert sein kann durch Fluor, Chlor, Brom, Jod, Alkyl mit 1 bis 4 Kohlenstoffatomen, Alkoxy mit 1 bis 4 Kohlenstoffatomen und/oder Phenyl, oder

Y für einen Rest der Formel 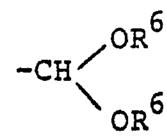 oder

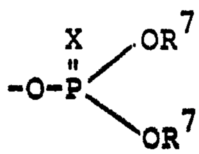

steht, worin

$R^6$ für Alkyl mit 1 bis 2 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 3 Kohlenstoffatomen steht und

X für Sauerstoff oder Schwefel steht.

4. Verfahren zur Herstellung von substituierten Diphenylethern der Formel

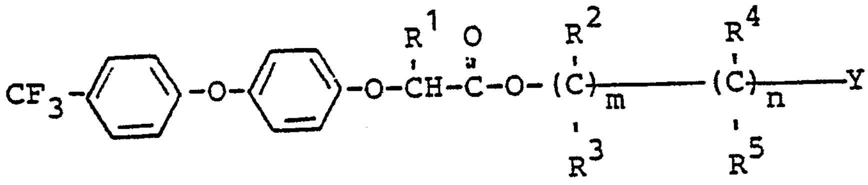

(I)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes, über Stickstoff gebundenes Azolyl und die Reste der Formeln

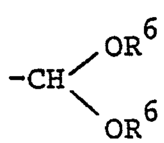 oder 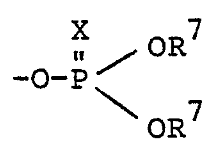 steht,

in welchen

$R^6$ für Alkyl mit 1 bis 4 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 4 Kohlenstoffatomen steht und

X für Sauerstoff oder Schwefel steht,

dadurch gekennzeichnet, daß man

a) zur Synthese derjenigen substituierten Diphenylether der Formel (I), in denen Y für Trimethylsilyl oder für den Rest der Formel

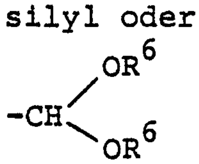 steht,

Phenoxyalkancarbonsäuren der Formel

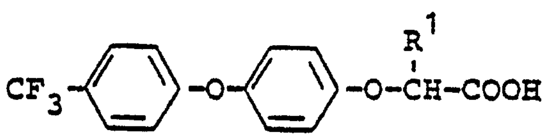 (II)

in welcher

$R^1$ die oben angegebene Bedeutung hat,

α) mit Silylchloriden der Formel

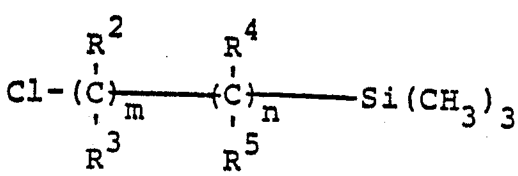 (IIIa)

in welcher

$R^2$, $R^3$, $R^4$, $R^5$, m und n die oben angegebene Bedeutung haben,

oder

β) mit Acetalen der Formel

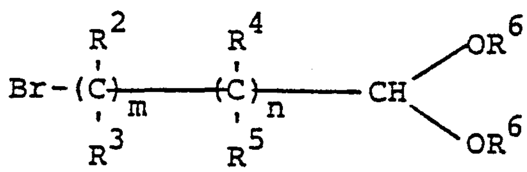

(IIIb)

in welcher

$R^2$, $R^3$, $R^4$, $R^5$, $R^6$, m und n die oben angegebene Bedeutung haben,

jeweils in Gegenwart eines Säurebindemittels sowie gegebenenfalls in Gegenwart eines Verdünnungsmittels umsetzt,

b) zur Herstellung derjenigen substituierten Diphenylether der Formel (I), in denen

Y für gegebenenfalls substituiertes über Stickstoff gebundenes Azolyl oder für die Reste der Formeln $-CH(OR^6)_2$ oder

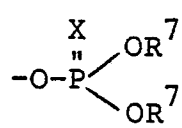

steht,

Phenoxyalkancarbonsäurechloride der Formel

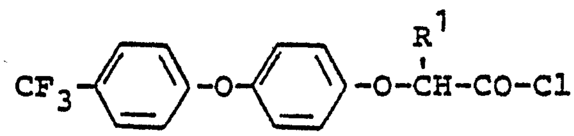
(IV)

in welcher

$R^1$ die oben angegebene Bedeutung hat,

mit Verbindungen der Formel

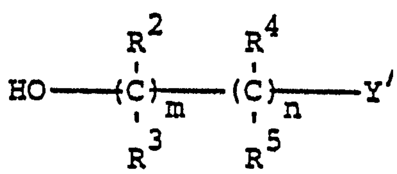
(V)

in welcher

$R^2$, $R^3$, $R^4$, $R^5$, m und n die oben angegebene Bedeutung haben und

Y' für gegebenenfalls substituiertes über Stickstoff gebundenes Azolyl, einen Rest der Formel $-CH(OR^6)_2$ oder der Formel

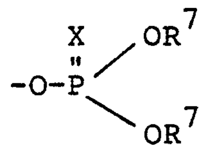

steht, wobei

$R^6$, $R^7$ und X die oben angegebene Bedeutung haben,

gegebenenfalls in Gegenwart eines Säurebindemittels sowie gegebenenfalls in Gegenwart eines Verdünnungsmittels umsetzt,

oder

c) zur Herstellung von substituierten Diphenylethern der Formel (I)

Benzotrifluoride der Formel

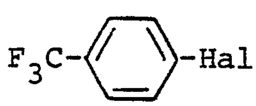

(VI)

in welcher

Hal für Chlor, Brom oder Jod steht,

mit Hydrochinon-monoethern der Formel

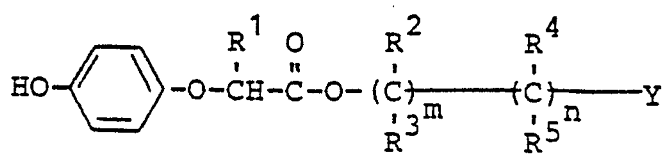

(VII)

in welcher

$R^1$, $R^2$, $R^3$, $R^4$, $R^5$, Y, m und n die oben angegebene Bedeutung haben,

gegebenenfalls in Gegenwart eines Säurebindemittels und gegebenenfalls in Gegenwart eines Verdünnungsmittels umsetzt,

oder

d) zur Herstellung von substituierten Diphenylethern der Formel (I)

4-(4-Trifluormethyl-phenoxy)-phenol der Formel

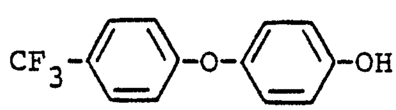

(VIII)

mit Alkancarbonsäure-Derivaten der Formel

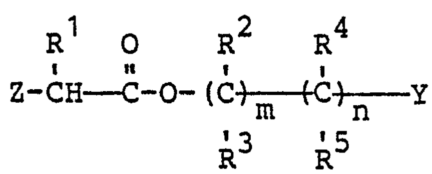

(IX)

in welcher

$R^1$, $R^2$, $R^3$, $R^4$, $R^5$, Y, m und n die oben angegebene Bedeutung haben und

Z für Chlor, Brom, Tosylat oder Mesylat steht,

gegebenenfalls in Gegenwart eines Säurebindemittels sowie gegebenenfalls in Gegenwart eines Verdünnungsmittels umsetzt.

5. Herbizide Mittel, gekennzeichnet durch einen Gehalt an mindestens einem substituierten Diphenylether der Formel (I).

6. Verwendung von substituierten Diphenylethern der Formel (I) zur Bekämpfung von Unkräutern.

7. Hydrochinon-monoether der Formel

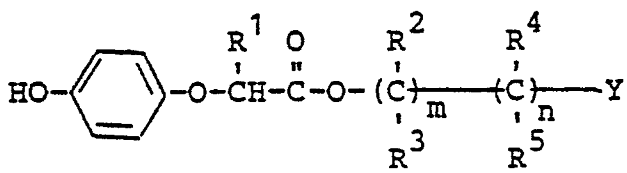

(VII)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes, über Stickstoff gebundenes Azolyl und die Reste der Formeln

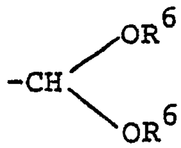 oder 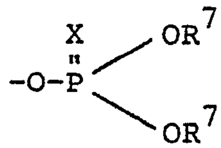 steht,

in welchen

$R^6$ für Alkyl mit 1 bis 4 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 4 Kohlenstoffatomen steht und

X für Sauerstoff oder Schwefel steht.

8. Verfahren zur Herstellung von Hydrochinon-monoethern der Formel

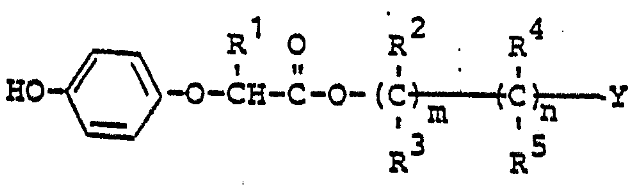 (VII)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes über Stickstoff gebundenes Azolyl und die Reste der Formeln

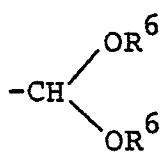 oder 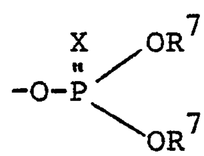 steht,

in welchen

$R^6$ für Alkyl mit 1 bis 4 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 4 Kohlenstoffatomen steht und

X für Sauerstoff oder Schwefel steht,

dadurch gekennzeichnet, daß man Hydrochinone der Formel

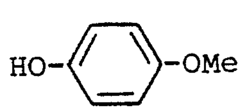 (XII)

in welcher

Me für Wasserstoff, Natrium, Kalium oder ein Äquivalent eines Calciummetalles steht,

mit Alkancarbonsäure-Derivaten der Formel

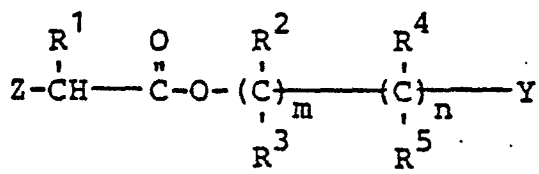

(IX)

in welcher

$R^1$, $R^2$, $R^3$, $R^4$, $R^5$, Y, m und n die oben angegebene Bedeutung haben

und

Z für Chlor, Brom, Tosylat oder Mesylat steht,

gegebenenfalls in Gegenwart eines Säurebindemittels sowie gegebenenfalls in Gegenwart eines polaren Verdünnungsmittels umsetzt.

9. Alkancarbonsäure-Derivate der Formel

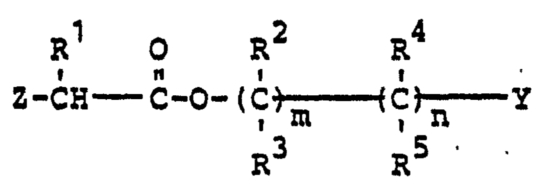

(IX)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes über Stickstoff gebundenes Azolyl und die Reste der Formeln

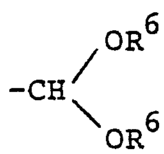 oder 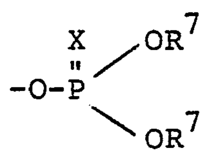 steht,

in welchen

$R^6$ für Alkyl mit 1 bis 4 Kohlenstoffatomen steht oder die beiden Reste $R^6$ gemeinsam für eine Alkylenkette mit 2 oder 3 Kohlenstoffatomen stehen,

$R^7$ für Alkyl mit 1 bis 4 Kohlenstoffatomen steht und

X für Sauerstoff oder Schwefel steht,

und

Z für Chlor, Brom, Tosylat oder Mesylat steht.

10. Verfahren zur Herstellung von Alkancarbonsäure-Derivaten der Formel

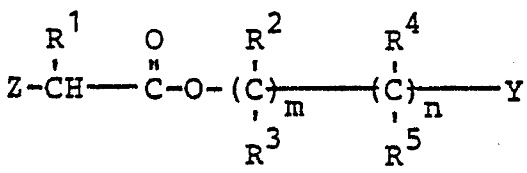

(IX)

in welcher

$R^1$ für Wasserstoff oder Methyl steht,

$R^2$, $R^3$, $R^4$ und $R^5$ unabhängig voneinander für Wasserstoff oder Alkyl mit 1 bis 4 Kohlenstoffatomen stehen,

m für 1 oder 2 steht,

n für 0 oder 1 steht und

Y für Trimethylsilyl, gegebenenfalls substituiertes über Stickstoff gebundenes Azolyl und die Reste der Formeln

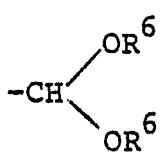 oder 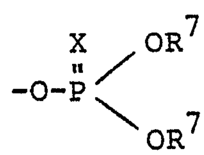 steht,

in welchen

$R^6$ für Alkyl mit 1 bis 4 Kohlenstoffatomen steht oder die beiden Reste $R^6$ gemeinsam für eine

Alkylenkette mit 2 oder 3 Kohlenstoffatomen stehen,

$R^7$ für Alkyl mit 1 bis 4 Kohlenstoffatomen steht und

X für Sauerstoff oder Schwefel steht,

und

Z für Chlor, Brom, Tosylat oder Mesylat steht,

dadurch gekennzeichnet, daß man Verbindungen der Formel

e)

$$R^1-CH_2-\overset{\overset{O}{\parallel}}{C}-Z^1 \qquad \text{(XIII)}$$

in welcher

$R^1$ die oben angegebene Bedeutung hat und

$Z^1$ für Hydroxy oder Chlor steht,

mit Verbindungen der Formeln

$$Cl-(\underset{R^3}{\overset{R^2}{C}})_m-(\underset{R^5}{\overset{R^4}{C}})_n-Si(CH_3)_3 \qquad \text{(IIIa)}$$

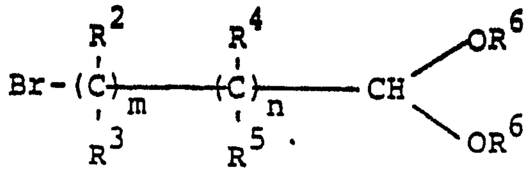

(IIIb)

oder

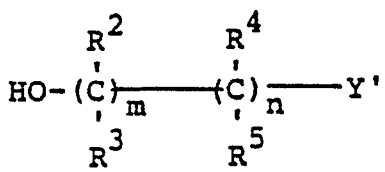

(V)

in welchen

$R^2$, $R^3$, $R^4$, $R^5$, $R^6$, Y', m und n die oben angegebenen Bedeutungen haben,

gegebenenfalls in Gegenwart eines Säurebindemittels sowie gegebenenfalls in Gegenwart eines Verdünnungsmittels umsetzt und die dabei entstehenden Stoffe der Formel

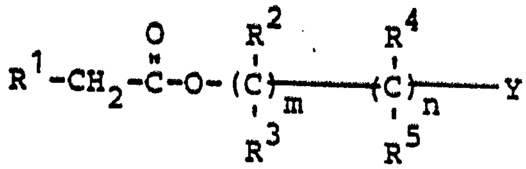

(XIV)

in welcher

$R^1$, $R^2$, $R^3$, $R^5$, $R^5$, Y, m und n die oben angegebenen Bedeutungen haben,

nach üblichen Methoden in die Alkancarbonsäure-Derivate der Formel (IX) überführt,

oder

f) indem man Verbindungen der Formel

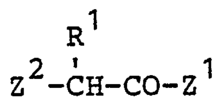

(XV)

in welcher

$R^1$ und $Z^1$ die oben angegebene Bedeutung haben und

$Z^2$ für Mesylat oder Tosylat steht,

mit Verbindungen der Formeln

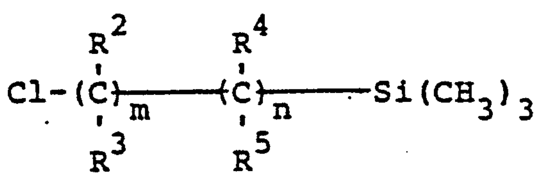

(IIIa)

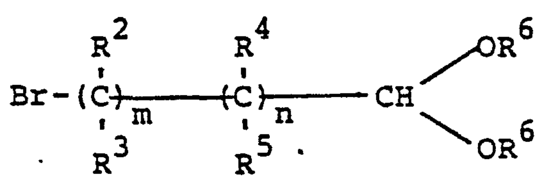

(IIIb)

oder

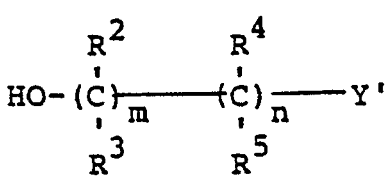

(V)

in welchen

$R^2$, $R^3$, $R^4$, $R^5$, $R^6$, Y', m und n die oben angegebenen Bedeutungen haben,

gegebenenfalls in Gegenwart eines Säurebindemittels sowie gegebenenfalls in Gegenwart eines Verdünnungsmittels umsetzt.

Europäisches Patentamt

**EUROPÄISCHER RECHERCHENBERICHT**

0095115
Nummer der Anmeldung

EP 83104785.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X | DE - A1 - 2 758 002 (HOECHST AG)<br>* Anspruch 1; Formeln I,II; Ansprüche 2-4 *<br>-- | 1,4-7, 9 | C 07 D 231/12<br>C 07 D 233/60<br>C 07 D 317/24<br>C 07 D 319/06<br>C 07 D 249/04<br>C 07 D 249/08<br>C 07 F 7/08<br>C 07 F 9/09<br>C 07 C 69/712<br>C 07 F 9/165<br>C 07 C 69/63<br>C 07 C 143/68<br>A 01 N 55/00<br>A 01 N 57/10<br>A 01 N 37/40<br>A 01 N 43/00 |
| D,A | DE - A1 - 2 223 894 (FARBWERKE HOECHST AG)<br>* Anspruch 1; Seiten 3,4 *<br>-- | 1,4-7 | |
| A | DE - A1 - 2 842 759 (MERCK PATENT GMBH)<br>* Ansprüche 1,3 *<br>-- | 1,4 | |
| A | DE - A1 - 2 938 534 (BAYER AG)<br>* Ansprüche 1,2 *<br>-- | 1,4 | RECHERCHIERTE SACHGEBIETE (Int. Cl. 3) |
| A | DE - A - 1 518 687 (CIBA AKTIEN GESELLSCHAFT)<br>* Ansprüche 1,5; Seiten 5-9 *<br>---- | 1,4-6 | C 07 D 231/00<br>C 07 D 233/00<br>C 07 D 317/00<br>C 07 D 319/00<br>C 07 D 249/00<br>C 07 F 7/00<br>C 07 F 9/00<br>C 07 C 69/00<br>C 07 C 143/00 |

Der vorliegende Recherchenbericht wurde für alle Patentansprüche erstellt.

| Recherchenort | Abschlußdatum der Recherche | Prüfer |
|---|---|---|
| WIEN | 23-08-1983 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument